## Europäisches Patentamt
## European Patent Office
## Office européen des brevets

(19)

(11) Veröffentlichungsnummer: **0 100 770**
**B1**

(12)

# EUROPÄISCHE PATENTSCHRIFT

(45) Veröffentlichungstag der Patentschrift:
**19.02.86**

(51) Int. Cl.⁴: **A 61 M 11/00** // B05B17/06, F24F6/12

(21) Anmeldenummer: **82107112.3**

(22) Anmeldetag: **06.08.82**

(54) **Vorrichtung zum Erzeugen eines Aerosols.**

(43) Veröffentlichungstag der Anmeldung:
**22.02.84 Patentblatt 84/8**

(45) Bekanntmachung des Hinweises auf die Patenterteilung:
**19.02.86 Patentblatt 86/8**

(84) Benannte Vertragsstaaten:
**AT BE CH DE FR GB IT LI LU NL SE**

(56) Entgegenhaltungen:
**DE - A - 2 941 554**
**FR - A - 2 328 451**

(73) Patentinhaber: **Klarhorst, Günter, Eisgrundstrasse 7, D-4800 Bielefeld 18 (DE)**
Patentinhaber: **Vaillant, Michael, Kleppingstrasse 9-11, D-4600 Dortmund 1 (DE)**

(72) Erfinder: **Klarhorst, Günter, Eisgrundstrasse 7, D-4800 Bielefeld 18 (DE)**

(74) Vertreter: **Schirmer, Siegfried, Osningstrasse 10, D-4800 Bielefeld 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft eine Vorrichtung zum Erzeugen eines Aerosols mit einer eine Flüssigkeit mittels eines Schwingungserzeugers zerstäubenden und das Aerosols abgebenden Vernebelungskammer un einem von einem Motor angetriebenen, in einer gesonderten Kammer untergebrachten Lüfterrad, welches Luft über ein Bakterienfilter ansaugt und die angesaugte Luft über eine gesonderte Verbindungsleitung dem oberen Teil der Vernebelungskammer zuführt, wobei das Lüfterrad über eine durch eine zwischen Motor und Lüfterrad angeordnete Trennwand hindurchwirkende Magnetkupplung mit dem Motor verbunden ist.

Ein Aerosol ist ein Gas, insbesondere Luft, das feste oder flüssige Schwebstoffe in feinstverteilter Form enthält. Aerosole aus Wasser und wässerigen Lösungen mit Tröpfchengrössen von 1 bis 5 µm werden häufig in der Medizin verwendet, wobei den wässerigen Lösungen zur Herstellung des Aerosols wasserlösliche Medikamente zugegeben werden können. Die in der Medizin verwendeten Aerosole aus Wasser und wässerigen Lösungen dienen unter anderem zur Erhöhung der Luftfeuchtigkeit in Operationsräumen und Intensivstationen und zur Befeuchtung des Atmungstraktes von patienten. Es ist hierbei wesentlich, dass die Aerosole keimfrei sind, um eventuelle Infektionen zu vermeiden. Keimfreie Aerosole lassen sich jedoch nur mit Vorrichtungen erzielen, die selbst keimfrei arbeiten.

Eine Vorrichtung der aufgezeigten Gattung ist aus der DE-B Nr. 2843756 bekannt. Bei dieser Vorrichtung ist das Lüfterrad in einer auswechselbaren Kassette untergebracht, die in einem Führungsrahmen des Gehäuses verschiebbar geführt und arretierbar ist. Mit Hilfe von Blattfedern und Einbuchtungen ist diese Kassette in ihrer Funktionsstellung festlegbar. Der Eintrittsöffnung der Lüfterradkammer ist ein Bakterienfilter vorgelagert, das zur Vermeidung des Ansaugens von Luft unter Umgehung des Bakterienfilters dicht angeschlossen sein muss.

Das in der Ansaugöffnung zum Lüfterrad angeordnete Bakterienfilter kann nicht verhindern, dass sich im Lüfterrad und im umgebenden Raum Krankheitserreger ansammeln, die auf dem Weg über die Vernebelungskammer in das Aerosol gelangen und eine Infektionsgefahr darstellen. Diese Gefahr ist vor allem dann gegeben, wenn das Bakterienfilter nicht dicht angeschlossen ist. Eine öftere Autoklavierung der Lüfterkassette und des Bakterienfilters ist erforderlich. Hierzu muss zunächst das Bakterienfilter seitlich aus dem Gehäuse und anschliessend die Kassette nach oben aus dem Gehäuse herausgezogen werden.

Damit die gesamte Vorrichtung stets voll funktionsfähig ist, müssen drei Einzelteile, nämlich die Vernebelungskammer, die Lüfterradkassette und das Bakterienfilter jeweils entfernt und in einem Autoklaven oder einem Sterilisator steril gemacht werden. Diese Arbeit ist zeitaufwendig und erfordert grösste Sorgfalt. Eine Gewähr dafür, dass alle drei Einzelteile stets einwandfrei steril gemacht und voll funktionsfähig wieder eingebaut worden sind, gibt es nicht. Es bleibt überwiegend der Sorgfalt des entsprechenden Personals überlassen, dass alle Teile steril und ordnungsgemäss wieder eingebaut werden. Eine Regulierung der Luftzuführung ist bei der bekannten Vorrichtung nicht möglich.

Aus der DE-A Nr. 2941554 ist des weiteren eine auswechselbare sterilisierbare Luftkassette für eine Vorrichtung zur Erzeugung eines Aerosols bekannt, bei der das Bakterienfilter und das Lüfterrad zu einer Baueinheit zusammengeschlossen sind. Bei dieser Ausführung ist das Bakterienfilter auswechselbar in einer Halterung der Kassette angeordnet. Die Luftkassette ist in das Gehäuse der Vorrichtung einschiebbar, wobei die angesaugte Luft über einen gesonderten flexiblen Schlauch, der ausserhalb des Gehäuses verläuft, der Verneblerkammer zugeführt wird. Eine volle Funktionsfähigkeit ist nur dann gegeben, wenn die Luftkassette ordnungsgemäss in das Gehäuse der Vorrichtung eingeschoben ist.

Bei dieser Ausführung müssen zwar nur noch zwei Hauptteile, nämlich die Luftkassette und die Verneblerkammer, einen Autoklaven oder einem Sterilisator zugeführt werden, jedoch sind durch das Erfordernis eines flexiblen Zuführschlauches zwischen Luftkassette und Verneblerkammer zusätzliche Sammelpunkte für Krankheitserreger gegeben, die unter Umständen in das Aerosol gelangen können. Ausserdem ist jeweils für einen ordnungsgemässen Anschluss des Verbindungsschlauches Sorge zu tragen.

Der Erfindung liegt die Aufgabe zugrunde, eine Vorrichtung der aufgezeigten Gattung so auszubilden, dass die mit dem Ein- und Ausbau der Elemente zum Zwecke der Zuführung zum Autoklaven oder Sterilisator verbundenen handgriffe auf ein Minimum reduziert sind und die Gewähr dafür gegeben ist, dass die Vernebelungskammer, das Lüfterrad und das Bakterienfilter in der gleichen Qualität steril gemacht und nach dem Einbau stets voll funktionsfähig sind und die Luftzuführung zum oberen Teil der Vernebelungskammer vereinfacht sowie eine gleichmässigere Verteilung der zugeführten Luft erreicht wird bei stufenloser Regelbarkeit der Luftzuführung.

Diese Aufgabe wird erfindungsgemäss dadurch gelöst, dass die Vernebelungskammer, das Lüfterrad und das Bakterienfilter zu einer Baueinheit zusammengeschlossen und auf der Aussenseite des Gehäuses senkrecht auf der das Lüfterrad enthaltenden Kammer abnehmbar angeordnet sind, wobei die Filterkammer ausserhalb der Wandung der Lüfterradkammer umlaufend angeordnet und das Bakterienfilter als Ringfilter ausgebildet ist und zur Herstellung der Verbindung zwischen Filterkammer und Lüfterradkammer in der Wandung der Lüfterradkammer Luftzuführöffnungen mit einem zugeordneten verschiebbaren Verschlussring angeordnet sind, der mit Öffnungen versehen ist, die mit den Luftzuführöffnungen in der Wandung der Lüfterradkammer deckungsgleich angeordnet sind und in der Vernebelungskammer ein oder mehrere senkrecht verlaufende Luftkanäle zur Führung der angesaugten Luft in den oberen Teil der Vernebelungskammer angeordnet sind. Ausgestaltungen der Erfindung sind in den übrigen Unteransprüchen gekennzeichnet.

Durch die erfindungsgemässe Ausbildung muss jeweils nur ein Element steril gemacht werden. Ein Vergessen eines der Elemente bei der Zuführung zum Autoklaven oder Sterilisator ist ausgeschlossen. Wesentliche Vorteile ergeben sich aus der verkürzten Ein- und Ausbauzeit bei Gewährleistung einer einwandfreien Funktion. Durch die Möglichkeit, die Luftzuführöffnungen stufenlos bis auf 0 mittels des verschiebbaren Verschlussringes zu verkleinern, kann die Geschwindigkeit der Nebelzuführung geregelt werden. Ausserdem kann bei Wegfall der Luftzuführung die Vorrichtung als Respirator eingesetzt werden. Hierzu wird über ein Y-Anschlusselement eine Verbindung zu einem Behälter mit angereichertem Sauerstoff hergestellt, wobei der in der Vernebelungskammer entstehende Nebel durch den Sauerstoff mitgerissen wird und dadurch eine Feuchtigkeitsanreicherung des Sauerstoffs erfolgt.

Ein Ausführungsbeispiel der Erfindung ist in der Zeichnung dargestellt und wird im folgenden näher beschrieben. Es zeigen:

Fig. 1 einen schematischen Vertikalschnitt durch die wesentlichen Teile der Vorrichtung;

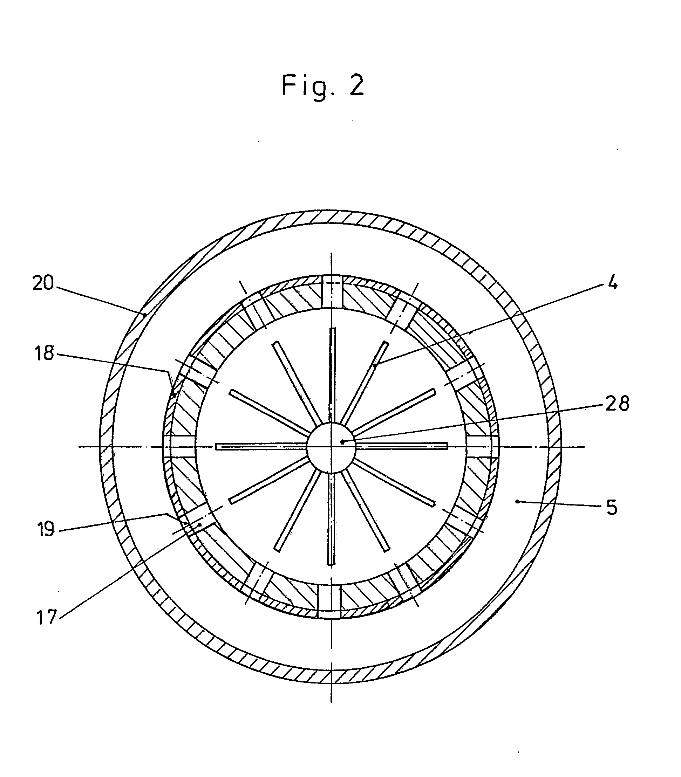

Fig. 2 einen Horizontalschnitt gemäss der Linie A-B, und

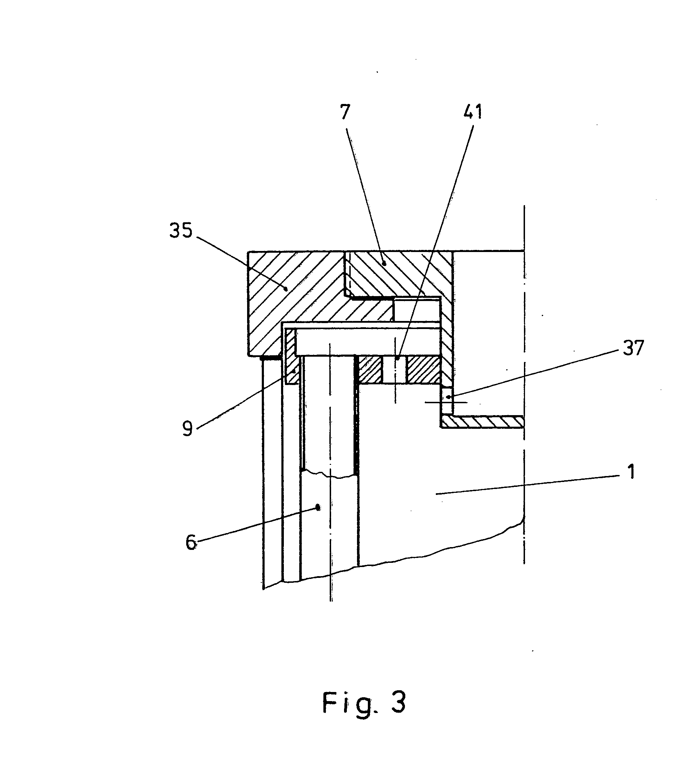

Fig. 3 eine vergrösserte Darstellung des Punktes A der Fig. 1 für eine Alternativlösung.

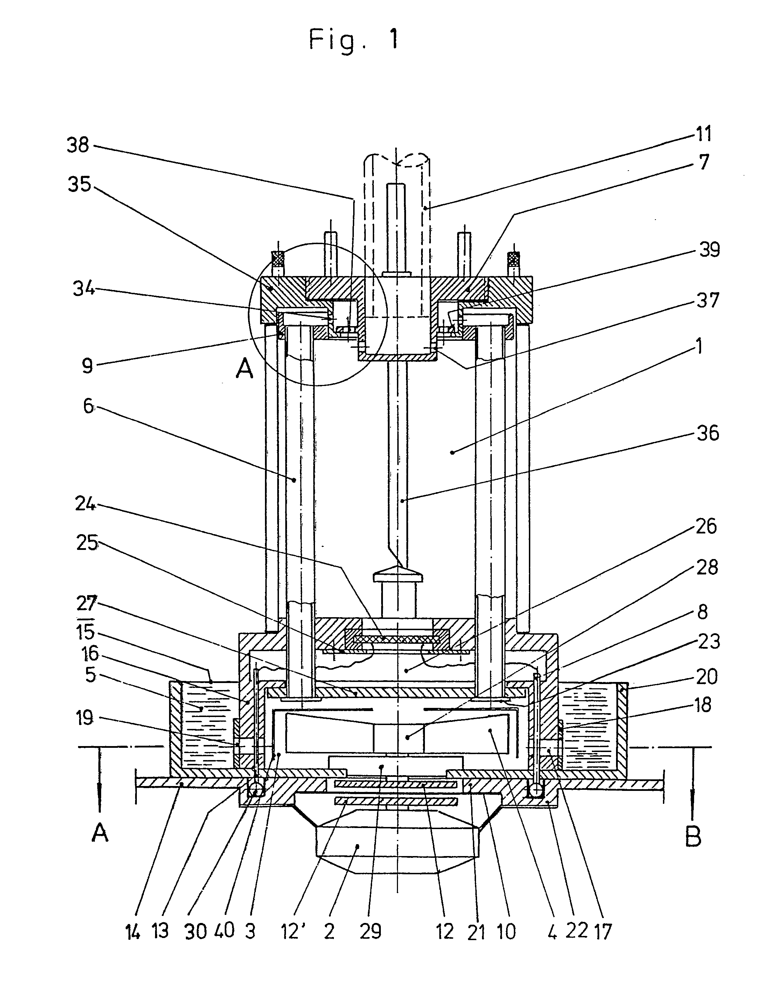

Wie aus Fig. 1 ersichtlich, ist auf dem Gehäuse 14 der Vorrichtung eine Lüfterradkammer 3 zur Aufnahme des Lüfterrades 4 angeordnet. Die Wandung 16 der Lüfterradkammer 3 ist über die Lüfterradkammer 3 hinaus nach oben verlängert und dient sowohl zur, seitlichen Begrenzung der Schwingunskammer 26, als auch zur Aufnahme der Vernebelungskammer 1, die durch einen aufgesetzten Glaszylinder gebildet ist. Ausserhalb der Wandung 16 der Lüfterradkammer 3 ist eine umlaufende Filterkammer 15 angeordnet, in der ein als Ringfilter ausgebildeter Bakterienfilter 5 auswechselbar lagert. Die Vernebelungskammer 1 mit dem Lüfterrad 4 und die Filterkammer 15 mit dem eingelegten Bakterienfilter 5 bilden eine Baueinheit, die mittels einer Steckverbindung 13 an das Gehäuse 14 angeschlossen sind.

In der Wandung 16 der Lüfterradkammer 3 sind in gleichmässigen Abständen auf dem Umfang verteilt Luftzuführöffnungen 17 angeordnet. Zur Regelung der Luftzuführung dient ein verschiebbarer Verschlussring 18, der um die Luftzuführöffnungen 17 verlaufend angeordnet ist. Im Verschlussring 18 sind Öffnungen 19 vorhanden, die mit den Luftzuführöffnungen 17 in der Wandung 16 der Lüfterradkammer 3 deckungsgleich sind. Zweckmässigerweise ist die Filterkammer 15 nach oben offen, wobei die Filterkammerwand 20 annähernd in Höhe des Bodens 27 der Schwingungskammer 26 endet.

Die Zerstäubung der Flüssigkeit erfolgt in der Vernebelungskammer 1 mit Hilfe des Ultraschall-Zerstäubereffektes, wobei die Flüssigkeit zu longitudinalen Schwingungen angeregt wird. Als Ultraschallschwingungserreger dient eine Quarzscheibe 24 mit Haltering 25, die an der unteren Begrenzung der Vernebelungskammer 1 in einer Schwingungskammer 26 angeordnet ist. Die zu zerstäubende Flüssigkeit kann Wasser oder eine wässerige Lösung mit wasserlöslichen Medikamenten sein. An die Vernebelungskammer 1 ist ein Abgabeschlauch 11 angeschlossen, aus welchem das Aerosol austritt und ggf. dem Atmungsorgan eines Patienten zugeführt wird. Mit oder auch ohne Abgabeschlauch 11 kann das Aerosol auch der Umgebungsluft zugeführt werden. Die Verwendung der erfindungsgemässen Vorrichtung ist nicht auf den medizinischen Sektor begrenzt. Der Einsatz kann auch da erfolgen, wo eine Erhöhung der Luftfeuchtigkeit erwünscht oder erforderlich ist, z. B. in der Nahrungsmittelindustrie und bei der Lederverarbeitung, in Museen, Bibliotheken und EDV-Räumen. Ausserdem lassen sich in gewissen Grenzen auch optische Wirkungen erzielen.

Der Motor 2, der durch eine beliebige Energiequelle antreibbar ist, z. B. durch eine Batterie, ist gemäss Fig. 1 durch eine Sicherheitsfolie 10 luft- und wasserdicht von der Lüfterradkammer 3 getrennt. Die Sicherheitsfolie 10 ist hierbei unterhalb einer umlaufenden Verstärkung 21 des Gehäuses 14 fest angeordnet. Die Luft- und wasserdichte Trennung kann auch durch das durchgehende Gehäuse 14 erfolgen, das an der entsprechenden Stelle eine Stärkereduzierung aufweist.

Die Verbindung zwischen Motor 2 und Lüfterrad 4 erfolgt über eine Magnetkupplung. Diese Magnetkupplung besteht aus den beiden Magnetscheiben 12 und 12'. Das Lüfterrad 4 ist ein Axiallüfter, welcher die Luft radial ansaugt und axial abgibt. Das Lager des Lüfterrades 4 ist mit 28 und die Lagerhalterung mit 29 bezeichnet.

In der Vernebelungskammer 1 sind zwei senkrecht verlaufende Luftkanäle 6 zur Zuführung der angesaugten Luft in den oberen Teil der Vernebelungskammer 1 angeordnet. Die Luftkanäle 6 sind luft- und wasserdicht unter Verwendung je eines Dichtungsringes 23 an den Boden der Schwingungskammer 26 angeschlossen. Am oberen Ende sind die Luftkanäle 6 an einem Halterungsring 9 angeschlossen, auf dem ein mit Öffnungen 34 versehener Verteiler- und Aufnahmering 35 für den Kammerdeckel 7 angeordnet ist, der zur Aufnahme des Abgabeschlauches 11 dient. Am Verteiler- und Aufnahmering 35 ist ein Niveaustandsröhrchen 36 angeordnet. Am Kammerdeckel 7 sind Austrittsöffnungen 37 vorgesehen. Diese Austrittsöffnungen 37 des Kammerdeckels 7 sind im Abstand von der oberen Begrenzung angeordnet, wobei oberhalb der Austrittsöffnungen 37 eine mit Lochungen 38 versehene Ring-Kragscheibe 39 vorgesehen ist. Der Deckel des Gehäuses 14 ist klappbar angeordnet.

Die Steckverbindung 13 ist durch senkrecht verlaufende elektrische Kontaktleitungen 8 gebildet, die an einem Kontaktring 30 angeschlossen sind, der in einer Ringwulst 22 des Gehäuses 14 lagert. Zur Vermeidung von Querluft kann zwischen der Filterkammer 15 und der Oberseite des Deckels des Gehäuses 14 eine Dichtungsscheibe angeordnet sein. Aus Fig. 1 ist eine von den Luftzuführöffnungen 17 zur Oberseite des Lüfterrades 4 führende Zwangsführung 40 für die angesaugte Luft ersichtlich.

Bei einer Alternativlösung sind im Halterungsring 9 umlaufend Durchbrechungen 41 zur Luftzuführung zur Vernebelungskammer 1 vorgesehen. Bei dieser Lösung können im Verteiler- und Aufnahmering 35 die Öffnungen 34 sowie die Anordnung der Ringkragscheibe 39 entfallen. Durch die Anordnung der Durchbrechungen 41 im Halterungsring 9 wird eine wesentlich gleichmässigere Luftverteilung und damit ein verbessertes Aerosol bei Wegfall einer Kondensatbildung an der Innenwandung der Vernebelungskammer 1 erreicht.

## Patentansprüche

1. Vorrichtung zum Erzeugen eines Aerosols mit einer eine Flüssigkeit mittels eines Schwingungserzeugers (24; 25) zerstäubenden und das Aerosol abgebenden Vernebelungskammer (1) und einem von einem Motor (2) angetriebenen, in einer gesonderten Kammer (3) untergebrachten Lüfterrad (4), welches Luft über ein in einer Filterkammer (15) auswechselbar angeordnetes Bakterienfilter (5) ansaugt und die angesaugte Luft über eine gesonderte Verbindungsleitung dem oberen Teil der Vernebelungskammer (1) zuführt, wobei das Lüfterrad (4) über eine durch eine zwischen Motor (2) und Lüfterrad (4) angeordnet Trennwand hindurchwirkende Magnetkupplung (12; 12') mit dem Motor (2) verbunden ist, dadurch gekennzeichnet, dass die Vernebelungskammer (1), das Lüfterrad (4) und das Bakterienfilter (5) zu einer Baueinheit zusammengeschlossen und auf der Aussenseite des Gehäuses (14) senkrecht auf der das Lüfterrad (4) enthaltenden Kammer (3) abnehmbar angeordnet sind, wobei die Filterkammer (15) ausserhalb der Wandung (16) der Lüfterradkammer (3) umlaufend angeordnet und das Bakterienfilter (5) als Ringfilter ausgebildet ist und zur Herstellung der Verbindung zwischen Filterkammer (15) und Lüfterradkammer (3) in der Wandung (16) der Lüfterradkammer (3) Luftzuführöffnungen (17) mit einem zugeordneten verschiebbaren Verschlussring (18) angeordnet sind, der mit Öffnungen (19) versehen ist, die mit den Luftzuführöffnungen (17) in der Wandung (16) der Lüfterradkammer (3) deckungsgleich angeordnet sind und in der Vernebelungskammer (1) ein oder mehrere senkrecht verlaufende Luftkanäle (6) zur Führung der angesaugten Luft in den oberen Teil der Vernebelungskammer (1) angeordnet sind.

2. Vorrichtung nach Anspruch 1, dadurch gekennzeichnet, dass die zu einer Baueinheit zusammengeschlossenen Teile (1; 4; 5) über eine Steckverbindung (13) an das Gehäuse (14) anschliessbar sind.

3. Vorrichtung nach Anspruch 1 oder 2, dadurch gekennzeichnet, dass die Luftzuführöffnungen (17) in gleichmässigen Abständen angeordnet sind.

4. Vorrichtung nach einem der Ansprüche 1 bis 3, dadurch gekennzeichnet, dass das Lüfterrad (4) ein Axiallüfter ist.

5. Vorrichtung nach einem der Ansprüche 1 bis 4, dadurch gekennzeichnet, dass zum Antrieb des Motors (2) eine Batterie angeordnet ist.

6. Vorrichtung nach einem der Ansprüche 1 bis 5, dadurch gekennzeichnet, dass die luft- und wasserdichte Trennwand zwischen Motor (2) und Lüfterradkammer (3) durch eine Sicherheitsfolie (10) gebildet ist.

7. Vorrichtung nach Anspruch 6, dadurch gekennzeichnet, dass die Sicherheitsfolie (10) unterhalb einer umlaufenden Verstärkung (21) des Gehäuses (14) fest angeordnet ist.

8. Vorrichtung nach einem der Ansprüche 1 bis 7, dadurch gekennzeichnet, dass die Steckverbindung (13) durch senkrecht verlaufende elektrische Kontaktleitungen (8) gebildet ist, die an einem in einer im Gehäuse (14) angeordneten Ringwulst (22) lagernden Kontaktring (30) anschliessbar sind.

9. Vorrichtung nach einem der Ansprüche 1 bis 8, dadurch gekennzeichnet, dass unterhalb der Vernebelungskammer (1) der an sich bekannte Schwingungserzeuger (24; 25) in einer besonderen Schwingungskammer (26) angeordnet ist, dessen Boden (27) gleichzeitig den oberen Abschluss der Lüfterradkammer (3) bildet.

10. Vorrichtung nach einem der Ansprüche 1 bis 9, dadurch gekennzeichnet, dass die in der Vernebelungskammer (1) angeordneten Luftkanäle (6) unten luft- und wasserdicht unter Verwendung je eines Dichtungsringes (23) an den Boden (27) der Schwingungskammer (26) und oben an einem Halterungsring (9) angeschlossen sind.

11. Vorrichtung nach Anspruch 10, dadurch gekennzeichnet, dass im Halterungsring (9) Durchbrechungen (41) zur Vernebelungskammer (1) angeordnet sind.

12. Vorrichtung nach einem der Ansprüche 1 bis 11, dadurch gekennzeichnet, dass zwischen der Filterkammer (15) und der Oberkante des Deckels des Gehäuses (14) eine Dichtungsscheibe angeordnet ist.

13. Vorrichtung nach einem der Ansprüche 1 bis 12, dadurch gekennzeichnet, dass von den Luftzuführöffnungen (17) zur Oberseite des Lüfterrades (4) eine Zwangsführung (40) für die angesaugte Luft angeordnet ist.

## Claims

1. Device for producing an aerosol with a liquid by means of an oscillator (24; 25) which atomizes and an atomization chamber (1) which dispenses the aerosol and a fan impeller (4) driven by a motor (2) located in a separate chamber (3); this impeller draws air in through a bacteria filter (5) replaceably mounted in a filter chamber (15) and passes the air drawn in via a separate connecting line to the upper part of atomization chamber (1), the fan impeller (4) being connected to motor (2) by a magnetic clutch (12; 12') acting through a partition located between motor (2) and fan impeller (4), characterized by the fact that the atomization chamber (1), the fan impeller (4) and the bacteria filter (5) are combined to form an integral unit and are detachably mounted on the outside of housing (14) vertically on the chamber (3) containing the fan impeller (4), the filter chamber (15) being located outside and around the wall (16) of fan impeller chamber (3) and bacteria filter (5) taking the form of an annular filter and to establish connection between filter chamber (15) and fan impeller chamber (3), air inlet openings (17) are provided in the wall (16) of fan impeller chamber (3), as well as a sliding obturator ring (18) which is provided with openings (19) which coincide with the air inlet openings (17) in the wall (16) of fan impeller chamber (3), and in atomization chamber (1) one or more vertical air passages (6) are provided to pass the aspirated air into the upper part of atomization chamber (1).

2. Device in accordance with Claim 1, characterized by the fact that the components (1; 4; 5) combined to form one integral unit can be connected to housing (14) by means of a "plug-and-socket" type joint (13).

3. Device in accordance with Claim 1 or 2, characterized by the fact that the air inlet openings (17) are located at uniform intervals.

4. Device in accordance with one of Claims 1 to 3, characterized by the fact that the fan impeller (4) is an axial fan.

5. Device in accordance with one of Claims 1 to 4, characterized by the fact that a battery is fitted to drive motor (2).

6. Device in accordance with one of Claims 1 to 5, characterized by the fact that the air and watertight partition between motor (2) and fan impeller chamber (3) takes the form of a safety foil (10).

7. Device in accordance with Claim 6, characterized by the fact that the safety foil (10) is positively located underneath a reinforcement rim (21) of housing (14).

8. Device in accordance with one of Claims 1 to 7, characterized by the fact that the "plug-and-socket" type joint (13) is formed by vertical electric contact leads (8) which are connectable a contact ring (30) locating in an annular ridge (22) formed in housing (14).

9. Device in accordance with one of Claims 1 to 8, characterized by the fact that underneath atomization chamber (1) the known oscillator (24; 25) is located in a special oscillation chamber (26) whose base (27) at the same time forms the top cover of fan impeller chamber (3).

10. Device in accordance with one of Claims 1 to 9, characterized by the fact that the air passages (6) provided in atomization chamber (1) are connected underneath, so as to form an air and watertight seal using a seal ring (23) in each case, to the base (27) of oscillation chamber (26), and at the top to a locating collar (9).

11. Device in accordance with Claim 10, characterized by the fact that openings (41) to atomization chamber (1) are provided in locating collar (9).

12. Device in accordance with one of Claims 1 to 11, characterized by the fact that a seal washer is provided between filter chamber (15) and the top edge of the cover of housing (14).

13. Device in accordance with one of Claims 1 to 12, characterized by the fact that a deflector (40) is provided to direct the aspirated air from the air inlet openings (17) to the top of fan impeller (4).

## Revendications

1. Un dispositif pour la production d'un aérosol avec une chambre de pulvérisation (1) pulvérisant un liquide au moyen d'un générateur de vibration (24; 25) et d'une roue de ventilateur (4) disposée dans une chambre séparée (3) et entraînée par un moteur (2) qui aspire de l'air à travers un filtre bactérien échangeable (5) installé dans une chambre de filtre (15) et qui conduit l'air aspiré par l'intermédiaire d'un conduit de raccordement séparé à la partie supérieure de la chambre de pulvérisation (1), la roue de ventilateur (4) étant reliée au moteur (2) par un accouplement magnétique (12, 12') agissant à travers une paroi disposée entre le moteur (2) et la roue de ventilateur (4), caractérisé par ceci que la chambre de pulvérisation (1), la roue de ventilateur (4) et le filtre bactérien (5) constituent un composant unitaire et sont disposés d'une manière amovible sur le côté extérieur du boîtier (14) verticalement sur la chambre (3) qui contient la roue de ventilateur (4), la chambre de filtre (15) étant disposée circulairement à l'extérieur de la paroi (16) de la chambre de roue de ventilateur (3) et le filtre bactérien (5) étant réalisé sous forme d'un filtre circulaire et des orifices d'alimentation d'air (17) avec une bague de fermeture coulissante correspondante (18) étant disposés dans la paroi (16) de la chambre de la roue de ventilateur (3) pour réaliser la liaison entre la chambre de filtre (15) et la chambre de roue de ventilateur (3), la bague de fermeture (18) étant munie d'ouvertures (19) qui correspondent aux orifices d'alimentation d'air (17) dans la paroi (16) de la chambre de roue de ventilateur (3), un ou plusieurs canaux d'air (6) orientés verticalement étant disposés dans la chambre de pulvérisation (1) pour conduire l'air aspiré à la partie supérieure de la chambre de pulvérisation (1).

2. Dispositif conforme à la revendication 1, caractérisé par ceci que les parties formant un composant unitaire (1, 4, 5) peuvent être reliés au boîtier (14) par l'intermédiaire d'une prise à fiche (13).

3. Dispositif conforme à l'une des revendications 1 ou 2, caractérisé par ceci que les orifices d'alimentation d'air (17) sont disposés selon un écart régulier.

4. Dispositif conforme aux revendications 1 à 3, caractérisé par ceci que la roue de ventilateur (4) est un ventilateur axial.

5. Dispositif conforme aux revendications 1 à 4, caractérisé par ceci que l'on utilise une batterie pour l'entraînement du moteur (2).

6. Dispositif conforme aux revendications 1 à 4, caractérisé par ceci que la paroi de séparation étanche à l'eau et à l'air entre le moteur (2) et la chambre de roue de ventilateur (3) est constituée par une feuille de sécurité (10).

7. Dispositif conforme à la revendication 6, caractérisé par ceci que la feuille de sécurité (10) est fixée sous un renfort circulaire (21) du boîtier (14).

8. Dispositif conforme aux revendications 1 à 7, caractérisé par ceci que la prise à fiche (13) est constituée par des conducteurs électriques (8) orientés verticalement et que ceux-ci peuvent être raccordés à une bague de contact (30) située dans un bourrelet annulaire (22) disposé dans le boîtier (14).

9. Dispositif conforme aux revendications 1 à 8, caractérisé par ceci que le générateur de vibration (24, 25) en soi connu est logé dans une chambre vibratoire particulière (26) sous la chambre de pulvérisation (1) et que le fond (27) de cette chambre vibratoire (26) constitue en même temps l'extrémité supérieure de la chambre de roue de ventilateur (3).

10. Dispositif conforme aux revendications 1 à 9, caractérisé par ceci que les canaux d'air (6) disposés dans la chambre de pulvérisation (1) sont raccordés en bas d'une manière étanche à l'eau et à l'air en utilisant pour chacun d'eux une bague d'étanchéité (23) sur le fond (27) de la chambre vibratoire (26) et en haut à une bague de fixation (9).

11. Dispositif conforme à la revendication 10, caractérisé par ceci que dans la bague de fixation (9), on a réalisé des découpures (41) conduisant à la chambre de pulvérisation (1).

12. Dispositif conforme aux revendications 1 à 11, caractérisé par ceci qu'entre la chambre de filtre (15) et l'arête supérieure du couvercle du boîtier (14) se trouve disposé un joint d'étanchéité.

13. Dispositif conforme à l'une des revendications 1 à 12, caractérisé par ceci qu'un passage obligatoire (40) depuis les orifices d'alimentation d'air (17) jusqu'à la partie supérieure de la roue de ventilateur (4) est installé pour l'air aspiré.

## Fig. 1

Fig. 2

Fig. 3